(19) **Europäisches Patentamt / European Patent Office / Office européen des brevets**

(11) **EP 2 563 413 B1**

(12) **EUROPEAN PATENT SPECIFICATION**

(45) Date of publication and mention of the grant of the patent:
**13.09.2017   Bulletin 2017/37**

(21) Application number: **11719724.4**

(22) Date of filing: **29.04.2011**

(51) Int Cl.:
*A61L 15/20* (2006.01)      *A61L 15/26* (2006.01)
*A61L 15/34* (2006.01)

(86) International application number:
**PCT/US2011/034518**

(87) International publication number:
**WO 2011/137323 (03.11.2011 Gazette 2011/44)**

(54) **NONWOVEN HAVING DURABLE HYDROPHILIC COATING**

VLIES MIT BESTÄNDIGER HYDROPHILER BESCHICHTUNG

NONTISSÉ COMPRENANT UN REVÊTEMENT HYDROPHILE DURABLE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.04.2010   US 329839 P**

(43) Date of publication of application:
**06.03.2013   Bulletin 2013/10**

(73) Proprietor: **The Procter & Gamble Company Cincinnati, OH 45202 (US)**

(72) Inventors:
• **CATALAN, Kemal, Vatansever**
  **Cincinnati**
  **Ohio 45255 (US)**
• **SPITZMUELLER, Carolyn, Ann**
  **Cincinnati**
  **Ohio 45255 (US)**
• **BUHIRU, Jensen, Kiziri**
  **Mt. Healthy**
  **Ohio 45231 (US)**
• **CHENG, Calvin, Hoi Wung**
  **Cincinnati**
  **Ohio 45208 (US)**
• **TALLY, Amy, Lynn**
  **Cold Spring**
  **Kentucky 41076 (US)**

(74) Representative: **Borbach, Markus Procter & Gamble Service GmbH IP Department Frankfurter Strasse 145 61476 Kronberg im Taunus (DE)**

(56) References cited:
**EP-A1- 0 400 694      WO-A2-2004/069915**
**US-A1- 2009 117 793      US-A1- 2009 118 421**

**Description**

FIELD OF THE INVENTION

The present disclosure relates to a nonwoven substrate having a durable hydrophilic coating. The coated nonwoven substrate may be particularly, but not exclusively, useful as a topsheet or absorbent core component in a disposable absorbent article, such as a diaper (nappy), incontinence pad, bandage, catamenial napkin, continence training pant, or the like.

BACKGROUND OF THE INVENTION

Disposable absorbent articles such as diapers, training pants, adult incontinence products and feminine care products may be used to collect and retain excreta deposited thereon by the wearer.

Nonwoven fabrics made of synthetic fibers and/or natural fibers are commonly used in absorbent articles, for example, as topsheet material or as a core wrap to enclose the storage layer of the absorbent core. Such nonwoven fabrics are often hydrophobic. However, for many applications in hygiene products it may be preferable to have hydrophilic nonwovens. Therefore, the nonwoven fabric may be treated to increase its hydrophilicity. For example, a core wrap may be a nonwoven material which has been rendered hydrophilic and designed to contain the storage layer and provide structural integrity when the storage layer is wet or dry.

One method for rendering nonwoven fabrics hydrophilic is coating the surface of the nonwoven with hydrophilic surfactants. However, hydrophilic surfactants may be washed away from the surface of the nonwoven by a gush of liquid. As the surfactant is washed away, the hydrophilicity of the nonwoven may decrease. One route to avoiding wash off is to add more surfactant, so that not all of the surfactant is washed away at a first, or second, or subsequent gush. With this approach, the reduction in hydrophilicity can be limited. However, due to surfactant wash-off, the surface tension of the liquid, which was in contact with the nonwoven fabric, may also be reduced. This reduction may be undesirable, because it can cause increased liquid leakage from the absorbent article.

Another method to render a nonwoven fabric hydrophilic is applying corona and/or plasma treatment. Plasma is an ionized form of gas that can be obtained by ionizing a gas or liquid medium. Plasma treatment may be used for the treatment of organic and inorganic materials to promote adhesion between various materials. Polymers that have chemically inert surfaces with low surface energies may not engage well with bondings and adhesives. Thus, these surfaces may be treated to facilitate bonding the surface with other substrates, coatings, adhesives, or printing inks. In addition to facilitating coating, the higher surface energy imparted by the corona or plasma treatment may itself increase the hydrophilicity of the surface. However, corona and plasma treatments lead to low coating durability upon storage of the treated material, i.e., hydrophilicity decreases over time.

Recently, new coating compounds have been reported to directly bond to or encapsulate the fibers of a nonwoven fabric. It was believed that these coatings would present a more durable hydrophilic coating in that they seemed less likely than prior coatings to be washed off the nonwoven fabric or otherwise inactivated. However, these coatings may also be associated with diminishing hydrophilicity over time. US 2009/117793 discloses a diaper comprising: a hydrophobic nonwoven substrate; and a coating composition comprising an aminopolyether-modified trialkoxysilane and acetic acid disposed on the nonwoven substrate. Accordingly, there is a need for a hydrophilic coating for a nonwoven which is durable during storage, including storage at elevated temperatures; does not migrate or transfer easily when dry; is not easily washed off when wetted or when fluid passes through or is in contact with the nonwoven; achieves a fast liquid strike-through time, both initially and following multiple exposures to aqueous fluids or liquids; does not significantly lower the surface tension of the fluid to be absorbed; and is not easily abraded or rubbed off the surface.

SUMMARY OF THE INVENTION

An absorbent article comprises at least trace amounts of a mineral oil, a hydrophilic nonwoven substrate disposed adjacent to the mineral oil; and a coating composition comprising a polyether-modified trialkoxysilane disposed on the nonwoven substrate and the further features of claim 1.

BRIEF DESCRIPTION OF THE DRAWINGS

Figure 1 is a plan view of an exemplary diaper in a flat, uncontracted state, that is, shown without any elastic-induced contraction.
Figure 2 is a sectional view along 2-2 showing an exemplary absorbent core structure.

Figure 3 is a sectional view along 2-2 showing an alternate exemplary absorbent core structure.

Figure 4 is a graph of impedance products by type of hydrophilic coating.

Figure 5 is sectional view of an exemplary structure for an absorbent core.

Figure 6 is a schematic view of a stack of layers used for ToF-SIMS analysis.

Figure 7 is a graph showing the wettability of various substrates and coatings.

DETAILED DESCRIPTION OF THE INVENTION

"Absorbent article" refers to devices that absorb and contain body exudates, and, more specifically, refers to devices that are placed against or in proximity to the body of the wearer to absorb and contain the various exudates discharged from the body. Absorbent articles may include diapers, training pants, adult incontinence undergarments, feminine hygiene products, breast pads, care mats, bibs, wound dressing products, pet pads, and the like. As used herein, the term "body fluids" or "body exudates: includes, but is not limited to, urine, blood, vaginal discharges, breast milk, sweat, and fecal matter.

"Adjacent" is used in the ordinary sense to refer to objects which have a common endpoint or border, as well as objects which have at least partially overlapping or interfaced surfaces.

"Coating" includes coatings that completely cover a surface, or portion thereof (for example, continuous coatings, including those that form films on the surface), as well as coatings that may only partially cover a surface, such as those coatings that after drying leave gaps in coverage on a surface (for example, discontinuous coatings). In some embodiments, the coating forms at least one layer of continuous film on the surface which has been coated, and is substantially uniform. However, when the coatings described herein are described as being applied to a surface, it is understood that the coatings need not be applied to, or that they cover, the entire surface. For instance, the coatings will be considered as being applied to a surface even if they are only applied to modify a portion of the surface.

"Comprise," "comprising," and "comprises" are open ended terms, each specifying the presence of what follows, e.g., a component, but not precluding the presence of other features.

"Disposable" is used in its ordinary sense to mean an article that is disposed or discarded after a limited number of usage events over varying lengths of time, for example, less than about 20 events, less than about 10 events, less than about 5 events, or less than about 2 events.

"Diaper" refers to an absorbent article generally worn by infants and incontinent persons about the lower torso so as to encircle the waist and legs of the wearer and that is specifically adapted to receive and contain urinary and fecal waste. As used herein, the term "diaper" also includes "pants", which is defined below.

"Durable hydrophilic coating" means a composition that, when coated on a nonwoven, does not migrate along the surface of the nonwoven or coated surface nor transfer from one surface to an adjacent surface during storage or elevated temperatures as measured by an e" value of less than 0.002gsm when tested in accordance with the test method described below.

"Hydrophilic" describes fibers or surfaces of fibers which are wettable by aqueous fluids (for example, aqueous body fluids) deposited on these fibers. Hydrophilicity and wettability are typically defined in terms of contact angle, for example, with a nonwoven fabric. This is discussed in detail in the American Chemical Society publication entitled "Contact angle, wettability and adhesion", edited by Robert F. Gould (Copyright 1964). A fiber or surface of a fiber is said to be wetted by a fluid (i.e., to be hydrophilic) when either the contact angle between the fluid and the fiber, or its surface, is less than 90°, or when the fluid tends to spread spontaneously across the surface of the fiber, as both conditions are normally co-existing. Conversely, a fiber or surface of the fiber is considered to be hydrophobic if the contact angle is greater than 90° and the fluid does not spread spontaneously across the surface of the fiber.

"Inboard" and "outboard" refer to the position of an object relative to the longitudinal or lateral centerline of an absorbent article or a component of an absorbent article. A first object is inboard of a second object if the first object is nearer the longitudinal or lateral centerline than the second object. A first object is outboard of a second object if the first object is farther from the longitudinal or lateral centerline than the second object.

A "nonwoven" is a manufactured sheet, web, or batt of directionally or randomly oriented fibers, bonded by friction, cohesion, and/or adhesion, excluding paper and products which are woven, knitted, tufted, stitch-bonded incorporating binding yarns or filaments, or felted by wet-milling, whether or not additionally needled. The fibers may be of natural or man-made origin or a combination of natural and man-made fibers, and may be staple or continuous filaments or may be formed in situ. Commercially available fibers have diameters ranging from less than about 0.001 mm to more than about 0.2 mm and they come in several different forms: short fibers (known as staple, or chopped), continuous single fibers (filaments or monofilaments), untwisted bundles of continuous filaments (tow), and twisted bundles of continuous filaments (yarn). Nonwoven fabrics can be formed by many processes, such as airlaying, meltblowing, spunbonding, solvent spinning, electrospinning, and carding. The basis weight of nonwoven fabrics can be expressed in grams per square meter (gsm).

"Pant" or "training pant", as used herein, refer to disposable garments having a waist opening and leg openings designed for infant or adult wearers. A pant may be placed in position on the wearer by inserting the wearer's legs into the leg openings and sliding the pant into position about a wearer's lower torso. A pant may be preformed by many techniques including, but not limited to, joining together portions of the article using refastenable and/or non-refastenable bonds (e.g., seam, weld, adhesive, cohesive bond, fastener, etc.). A pant may be preformed anywhere along the circumference of the article (for example, side fastened, front waist fastened). While the terms "pant" or "pants" are used herein, pants are also commonly referred to as "closed diapers," "prefastened diapers," "pull-on diapers," "training pants," and "diaper-pants". Some exemplary pants are disclosed in U.S. Patent No. 5,246,433; U.S. Patent No. 5,569,234; U.S. Patent No. 6,120,487; U.S. Patent No. 6,120,489; U.S. Patent No. 4,940,464; U.S. Patent No. 5,092,861; U.S. Patent Publication No. 2003/0233082 A1, filed on June 13, 2002; U.S. Patent No. 5,897,545; and U.S. Patent No. 5,957,908.

"Trace amount," as used herein, refers to any detectable quantity of a substance, using any known method for detection.

Aspects of the present disclosure involve a durable hydrophilic coating for a nonwoven substrate, including components of a coating solution, processes for applying a coating solution according to the present disclosure, and substrates to which a coating solution may be applied. Without wishing to be bound by theory, a current understanding of some of the challenges in providing a durable hydrophilic coating is described. A nonwoven substrate coated with a coating solution as described herein may be useful in a variety of articles, including absorbent articles. To provide context, such as exemplary uses for a nonwoven substrate having a durable hydrophilic coating, the discussion below includes references to components of an exemplary absorbent article. The structure of an exemplary diaper is described in greater detail following the description of the coating solution components, processes, and substrates. A method for reducing the migration of a hydrophilic coating within an absorbent article is also described.

As mentioned above, one problem encountered when coating nonwovens to confer hydrophilicity is that some coatings can be washed away from the nonwoven when exposed to liquid. In the context of an absorbent article, liquid exposure may be referred to as a gush, event, or insult, and includes exposure to the fluid components of urine, feces, menses, and other body exudates. Another problem is that a functional surfactant, such as a hydrophilic coating, may migrate across or away from the nonwoven, even under dry conditions (i.e., when exposed to airborne humidity only, with no liquid-phase exposure as might be encountered during use).

Some coatings may fail due to "fouling" by compounds which are leached from other components of the diaper. In particular, the mineral oils and related compounds that are used as plasticizers in thermoplastic adhesives (such as hot melt adhesives), which may be used to construct disposable absorbent articles, can mobilize over time and interact with some hydrophilic coatings in a manner which compromises the function of the coating. This may occur even when the concentration of plasticizers in these adhesives is quite small, and even though the adhesive is solid after cooling (which made it seem unlikely that plasticizers would leach from the adhesives under typical storage conditions). Without wishing to be bound by theory, it is currently believed that the surfactants and/or wetting agents which may be used to help disperse hydrophilic coatings may also facilitate the leaching and spreading of plasticizers from adhesives which are relatively near the hydrophilic coating in the assembled product.

Some surfactants, including the active ingredient in many hydrophilic coatings, are soluble or miscible in semi- or non-polar solvents. Thus, when mineral oil contacts the coating, the surfactant can partition into the oil. Particularly, but not exclusively, where the surfactant is already liquid, the oil mixes with the surfactant, and has the potential to increase the mobility of the combined oil and surfactant mixture. In the case where the mobility of the combined oil and surfactant mixture is increased, the mixture can then form aggregates such as micelles, which continue to attract additional oils, such as additional plasticizers, oils from a lotion treatment, skin oils, and the like. The mixture itself may be mobile, and may remove hydrophilic compounds from the nonwoven and may transfer hydrophilic compounds to adjacent structures. For example, a non-aqueous mixture of the sort described above may move across a core cover, and transfer to the acquisition layer, barrier leg cuffs, and/or topsheet of an absorbent article. This mobility between structures may be pronounced when the absorbent article is stored in a compressed state, such that adjacent surfaces are in close contact for a period of weeks or months as the product is shipped and stored by a distributor, retailer, and/or user.

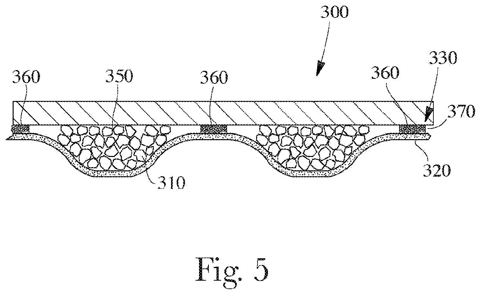

If this theory is correct, the problem of declining hydrophilicity over time may be exacerbated for absorbent articles with a core construction similar, at least in part, to that shown in FIG. 5. FIG. 5 shows an exemplary absorbent core component having sandwich structure 300 comprising absorbent material 310 sandwiched between carrier material 320 and cover material 330. Absorbent material 310 is formed into absorbent clusters 350 separated by bonding point regions 360. Channel regions 370 occur between neighboring bonding point regions 360. Each bonding point region 360 may comprise an adhesive, and additional adhesive may be used under, within, or on top of absorbent material 310 in each of absorbent clusters 350. Absorbent clusters 350 may be discontinuous along the absorbent core, in the longitudinal and/or transverse directions. A sheet such as that exemplified in Fig. 5 may be used singly, or two or more layers of sheets similar to that shown in Fig. 5 may be combined to produce an absorbent core. If multiple sheets are used, absorbent clusters 350 in each layer may be aligned or offset in the longitudinal and/or transverse directions.

Structures like that illustrated in Fig. 5 may serve to immobilize particulate or fibrous absorbent materials, particularly when wet. Such wet immobilization may prevent particulate or fibrous absorbent materials from migrating through the absorbent article and contacting the wearer's skin. Skin contact may be undesirable if the absorbent material might be irritating, might be perceived as a possible irritant, might be perceived as unhygienic, might be associated with cheap or poorly made articles, or might be considered aesthetically unacceptable. Wet immobilization may also help to preserve open channels or other routes for liquid flow through an absorbent sandwich structure, and thereby avoid the gel-blocking phenomenon encountered with some absorbent gelling materials. Exemplary variants of the structure shown in Fig. 5 are described, for example, in U.S. Patent Application Publication Nos. 2004/0167486, 2006/0021695, and 2008/0125735.

Because it may be difficult to find a cost-effective, processable adhesive that is free of plasticizers, these structures may exacerbate problems with declining hydrophilicity both because of the relatively large amount of adhesive used in the absorbent core and the proximity of the adhesive to hydrophilized nonwoven structures, such as topsheet 18 or absorbent core wrap 62, in addition to any adhesives which might be used to join various layers and/or components of an absorbent article with any core structure or absorbent articles having no core. Of course, mineral oils and related compounds from other sources may also foul the hydrophilic coating. For example, there may be plasticizers in elastic or film components, or oleaginous compounds in compositions applied to the absorbent article to promote skin health, prevent diaper rash, simplify clean-up (e.g., anti-stick compositions to prevent bowel movements or menses from adhering to skin or hair), and the like. Some oleaginous compounds may be introduced to the article during use, such as naturally occurring body oils, or lotions or creams that are applied to a wearer and transfer from the wearer to the article during use.

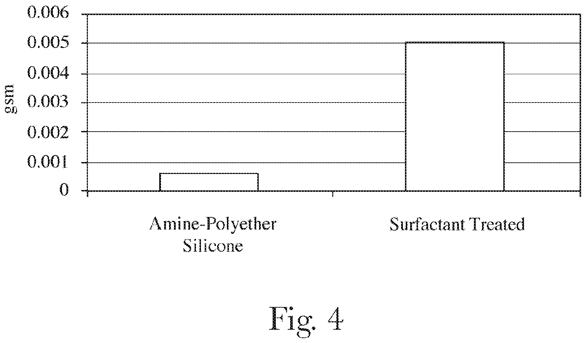

It has been shown that one particular sort of hydrophilic coating, specifically, an aminopolyether-modified trialkoxysilane coating, may be less prone to fouling than other coatings. The reason that this particular coating is less susceptible to fouling is unknown, however, FIG. 4 shows that the extractable active coating compound recovered from an adjacent, untreated nonwoven layer after three months of real-time (RT) aging may be significantly lower using the amine-polyether silicone of the present disclosure than using a known surfactant treatment, suggesting that the amine-polyether silicone treatment may be less prone to dry migration to adjacent structures. The aminopolyether-modified trialkoxysilane is believed to provide a durable coating through cross-linking siloxane groups that bond the coating to the surface of a nonwoven substrate, and alkoxy groups which confer hydrophilicity. There was no particular reason to believe that aminopolyether-modified trialkoxysilanes would significantly outperform other self-cross-linking hydrophilic coatings, such as UV grafting or plasma-induced coating, in terms of fouling (as measured by dry migration via Impedance and/or ToF-SIMS). Disclosed aminopolyether-modified trialkoxysilanes include, but are not limited to, polyether moieties having an epoxy endcapped polyether with the average structure $CH_2(O)CHCH_2(OCH_2CH_2)_{11.7}OCH_2CH(O)CH_2$. Other disclosed aminopolyether-modified trialkoxysilanes and methods of making them may include the hydrophilic compounds disclosed in U.S. Patent Application Publication No. 2009/0117793 to Falk et al., and U.S. Patent Application Publication No. 2009/0118421 to Falk. For example, disclosed aminopolyether-modified trialkoxysilanes may include polyether moieties of the formula:

$$R^{26}O(R^{27})_\gamma(C_2H_4O)_\delta(C_3H_6O)_{68}(C_4H_8)_\zeta R^{28}$$

where $R^{26}$ and $R^{28}$ are independently a monovalent hydrocarbon radical containing one or more oxirane or oxetane moieties having from 3 to 12 carbon atoms; $R^{27}$ is selected from the group of divalent radicals consisting of $-C_2H_4O-$, $-C_3H_6O-$, and $-C_4H_8O-$; the subscript $\gamma$ is zero or 1; the subscript $\delta$ is zero or positive and has a value ranging from 50 to about 100; the subscript $\varepsilon$ is zero or positive and has a value ranging from 0 to about 100; and the subscript $\zeta$ is zero or positive and has a value ranging from 0 to about 100 subject to the limitation that the molecule remains polar.

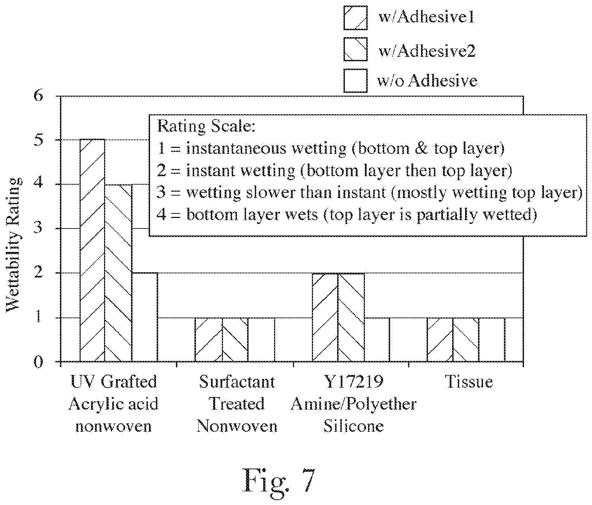

FIG. 7 is a graph showing the wettability rating of a nonwoven with three different treatments compared to tissue (two layers of nonwoven with no adhesive present). The wettability rating has a scale from 1 to 5, where 1 is best. The detailed scale is provided with the test method for wettability, below. As can be seen from the graph, the amine-polyether silicone treatment of the present disclosure, in the presence of adhesives, may produce wetting on par with UV grafted acrylic acid treatment in the absence of adhesives. Unlike UV grafted acrylic acid treatment, exposure of the amine-polyether silicone treatment of the present disclosure to adhesives may not impede wettability of the nonwoven substrate relative to tissue. That is, although the UV-crosslinked treatment showed evidence of fouling (reduced wettability), the aminopolyether-modified trialkoxysilane coating did not show significant evidence of fouling.

COATING SOLUTION AND PROCESS

When preparing a coating solution, it may be desirable to include coating solution components other than the aminopolyether-modified trialkoxysilanes to facilitate application and spreading of the coating in a desired manner. For example, it may be desirable to include a wetting agent to help distribute the aminopolyether-modified trialkoxysilanes across the surface of a nonwoven substrate. Although wetting agents in some coatings may exacerbate fouling by increasing the mobility of oleaginous compounds, the aminopolyether-modified trialkoxysilanes include terminal hydroxy groups which can interact with some wetting agents, chemically incorporating the wetting agent into the coating such that there is no free wetting agent available to wash off or migrate from the coated surface. Thus, is may be desirable to include a wetting agent having terminal hydroxy functional groups, to enable this incorporation into the amine-polyether silicone compound. A mixture of wetting agents may be used.

Non-limiting examples of wetting agents include nonionic and amphoteric surfactants such as Gemini diol ethoxylates, nonionic surfactants of Hydrophilic-Lipophilic Balance (HLB) between 3 and 16, the $C_{12}$-$C_{18}$ alkyl ethoxylates ("AE") including the narrow peaked alkyl ethoxylates and $C_6$-$C_{12}$ alkyl phenol alkoxylates (for example, ethoxylates and mixed ethoxy/propoxy), $C_{12}$-$C_{18}$ betaines and sulfobetaines ("sultaines"), $C_{10}$-$C_{18}$ amine oxides, and the like. In one embodiment, the wetting agent is a nonionic surfactant sold under the trade name "DYNOL 604" by Air Products and Chemical, Inc. In another embodiment, the wetting agent is a nonionic surfactant sold under the trade name "DYNOL 607" by Air Products and Chemical, Inc. In another embodiment, the wetting agent is a nonionic surfactant sold under the tradename SURFYNOL™, by Air Products and Chemical, Inc, for example, Surfynol 420, Surfynol 440, Surfynol 465, or Surfynol 485. In another embodiment, the wetting agent is a nonionic surfactant sold under the tradename CARBOWET, by Air Products and Chemical, Inc, e.g., Carbowet 106, or Carbowet 109. And, in another embodiment, the wetting agent is a nonionic surfactant sold under the tradename NEODOL, by Shell Chemicals, for example, Neodol 91-6, Neodol 23-3, Neodol 1-9, Neodol 1-7, Neodol 91-8, or Neodol 45-7.

Another class of useful wetting agents includes silicone surfactants and/or silicones. They can be used alone and/or alternatively in combination with the alkyl ethoxylate surfactants described above. Nonlimiting examples of silicone surfactants are the polyalkylene oxide polysiloxanes having a dimethyl polysiloxane hydrophobic moiety and one or more hydrophilic polyalkylene side chains, and having the general formula:

$$R^1\text{-}(CH_3)_2SiO\text{-}[(CH_3)_2SiO]_a\text{-}[(CH_3)(R^1)SiO]_b\text{-}Si(CH_3)_2\text{-}R^1$$

wherein a + b are from about 1 to about 50, and each $R^1$ is the same or different and is selected from the group consisting of methyl and a poly(ethyleneoxide/propyleneoxide) copolymer group having the general formula: $-(CH_2)_n O(C_2H_4O)_c (C_3H_6O)_d R^2$, wherein n is 3 or 4; total c (for all polyalkyleneoxy side groups) has a value of from 1 to about 100, alternatively from about 6 to about 100; total d is from 0 to about 14; alternatively d is 0; total c+d has a value of from about 5 to about 150, alternatively from about 9 to about 100 and each $R^2$ is the same or different and is selected from the group consisting of hydrogen, an alkyl having 1 to 4 carbon atoms, and an acetyl group, alternatively hydrogen and methyl group. Each polyalkylene oxide polysiloxane has at least one $R^1$ group being a poly(ethyleneoxide/propyleneoxide) copolymer group. Silicone wetting agents are available from Dow Corning as silicone glycol copolymers (for example, Q2-5211 and Q2-5212), from Degussa, sold under the tradename TEGOWET, and from Momentive Performance Materials, sold under the tradename Silwet. The coating composition includes and acid, which is acetic acid to hydrolyze the alkoxy groups from the silane, which may facilitate cross-linking and improve durability. It is disclosed that any acid could be used, and that citric acid may be convenient because of its relative strength and low odor during application. A defoamer may be desirable to facilitate transport and application of the coating solution.

The coating solution may be applied at any desired amount, for example, at least 0.1% weight of the coating solution to weight of the uncoated substrate. For use in a disposable absorbent article, it may be desirable to use a small enough amount of coating solution to avoid the formation of a noticeable film on the nonwoven substrate. This may be true, for example, where the coated nonwoven substrate will be used as a visible or wearer-contacting surface in the disposable absorbent article, such as a topsheet. It may be desirable to maintain a coating thickness smaller than the caliper or thickness of the nonwoven substrate.

The coating solution may be applied in a continuous fashion. For example, the coating solution may be applied to the substrate by immersing the substrate in the coating or otherwise covering all or substantially all of the surface area of at least one surface of the substrate. The coating solution may be applied in a discontinuous fashion, resulting in a continuous coating or a discontinuous coating. For example, the coating solution may be applied in stripes, in an amount and spacing that result in a continuous coating as the coating solution spreads over the surface of the substrate. Alternately, the coating solution may be applied in stripes, in a relatively smaller amount and/or sparser spacing, resulting in a discontinuous coating. Any coating pattern may be used, including, but not limited to, stripes, dots, geometric shapes, and irregular patterns. Decorative patterns may be used, however, a very thin coating and/or the presence of a wetting agent may limit the ability to maintain crisp "images" in the final coating. The coating solution may be applied using any desirable method, including, but not limited to, kissrolling, immersion, spraycoating, printing, etc. It may be desirable to adjust the coating method to obtain permeation of the coating through the substrate in a z-direction orthogonal to the plane defined by the longitudinal and lateral axes of the absorbent article.

The coating solution may be applied after, or immediately after, or concurrent with, a surface treatment to increase the surface energy of the substrate to be coated. For example, the substrate may be subject to atmospheric plasma treatment, corona treatment, chemical plasma treatment, or other high-energy surface treatments which modify the properties, more specifically, the surface energy and/or chemical reactivity, of a surface without changing the dimensions of the substrate. The dimension of the substrate is, for the purpose of the present disclosure, unchanged if there is no change in dimension at or above the order of micrometers-that is, there may be dimensional changes less than 1μm in magnitude. In some embodiments, no surface treatment is used. In some embodiments, a substrate may be subjected to one or more kinds of surface treatments at two or more times. For example, a substrate may be subjected to plasma treatment during manufacture of the substrate, and may be subjected to plasma treatment again during conversion into a final product. Hours, days, or weeks may elapse between the surface treatments if multiple surface treatments are used. The use of a surface treatment may allow the use of less coating solution on the substrate without a corresponding decrease in the effect of the coating.

SUBSTRATES

The coating described herein may be useful for hydrophilizing any type of hydrophobic nonwoven substrate. It has been shown that the coating may be useful, for example, with a spunbond nonwoven substrate, rendering the spunbond nonwoven substrate durably hydrophilic and suitable for use, for example, as an acquisition layer in a disposable absorbent article. If the nonwoven substrate is not inherently comprised of staple fibers, it may be desirable to include some staple fibers in the substrate. In some structures, the staple fibers may contribute to the structural integrity of the nonwoven substrate by providing a hydrophilic interconnection within the interstices of other fibers. Staple fibers disposed in the interstices of other fibers may also facilitate liquid transport by providing additional surface area for fluid flow through the nonwoven substrate.

ABSORBENT ARTICLES

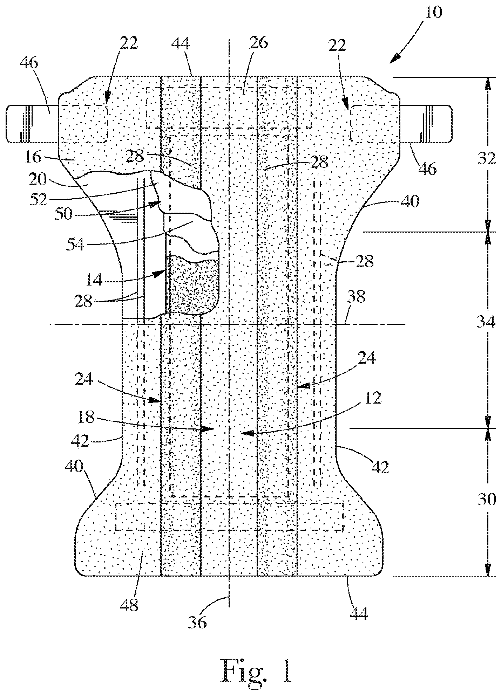

Fig. 1 is a plan view of an exemplary disposable absorbent article 10, such as a diaper, according to the present disclosure. The diaper 10 is shown in its flat out, uncontracted state (i.e., without elastic induced contraction) and portions of the diaper 10 are cut away to more clearly show the underlying structure of the diaper 10. A portion of the diaper 10 that contacts a wearer, that is, the body-facing surface, is facing the viewer in Fig. 1. The diaper 10 generally may comprise a chassis 12 and an absorbent core 14 disposed in the chassis.

The chassis 12 of the diaper 10 in Fig. 1 may comprise the main body of the diaper 10. The chassis 12 may comprise an outer covering 16 including a top sheet 18, which may be liquid pervious, and/or a back sheet 20, which may be liquid impervious. The absorbent core 14 may be encased between the top sheet 18 and the back sheet 20. The chassis 12 may also include side panels 22, elasticized leg cuffs 24, and/or an elastic waist feature 26. Topsheet 18, leg cuffs 24, and other body-contacting surfaces may include lotion or skin treatment compositions for imparting benefits to the skin of the wearer. Lotion or skin treatment compositions may include waxes, oils, or other oleaginous compounds.

The leg cuffs 24 and the elastic waist feature 26, if present, may comprise elastic members 28. One end portion of the diaper 10 may be configured as a first waist region 30 of the diaper 10. An opposite end portion of the diaper 10 may be configured as a second waist region 32 of the diaper 10. An intermediate portion of the diaper 10 may be configured as a crotch region 34, which extends longitudinally between the first and second waist regions 30 and 32. The waist regions 30 and 32 may include elastic elements such that they gather about the waist of the wearer to provide improved fit and containment, shown as elastic waist feature 26. The crotch region 34 is that portion of the diaper 10 which, when diaper 10 is worn, is generally positioned between the wearer's legs. In some embodiments, crotch region 34 may be shifted somewhat toward first or front waist region 30 or second or rear waist region 32, as, for example, in a diaper designed specifically for a male or female wearer. Crotch region 34 may extend longitudinally between about 25% and about 75% of the longitudinal length of diaper 10.

Diaper 10 is depicted in Fig. 1 with longitudinal axis 36 and transverse axis 38. Periphery 40 of the diaper 10 is defined by the outer edges of diaper 10 in which longitudinal edges 42 run generally parallel to longitudinal axis 36 and end edges 44 run generally parallel to transverse axis 38. Chassis 12 may also comprise a fastening system, which may include one or more fastening members 46 and/or one or more stored landing zones 48.

Diaper 20 may also include other features, including, but not limited to, front and rear ear panels, waist cap features, elastics and the like to provide better fit, containment and aesthetic characteristics. Some exemplary additional features are described, for example, in U.S. Pat. No. 3,860,003 and U.S. Pat. No. 5,151,092.

For unitary absorbent articles, chassis 12 and absorbent core 14 may form the main structure of diaper 10 with other features added to form the composite diaper structure. While topsheet 18, backsheet 20, and absorbent core 14 may be assembled in a variety of configurations, exemplary diaper configurations are described generally in U.S. Pat. No. 5,554,145; U.S. Pat. No. 5,569,234; and U.S. Pat. No. 6,004,306.

In some embodiments, diaper 10 may also comprise acquisition system 50 disposed between the liquid permeable top sheet 18 and a wearer facing side of absorbent core 14. Acquisition system 50 may be in direct contact with the absorbent core. Acquisition system 50 may comprise a single layer or multiple layers, such as an upper acquisition layer 52 facing the wearer's skin and a lower acquisition 54 layer facing the garment of the wearer. In some embodiments, acquisition system 50 may function to receive a surge of liquid, such as a gush of urine, and quickly absorb the liquid and distribute it across absorbent core 14 so that absorbent core 14 absorbs the liquid before the liquid flows beyond absorbent core 14 and out of diaper 10. In other words, acquisition system 50 may serve as a temporary reservoir for liquid until absorbent core 14 can absorb the liquid.

In some embodiments, acquisition system 50 may comprise chemically cross-linked cellulosic fibers. Such cross-linked cellulosic fibers may have desirable absorbency properties. Exemplary chemically cross-linked cellulosic fibers are disclosed in US Patent No. 5,137,537.

In some embodiments, one or both of upper and lower acquisition layers 52 and 54 may comprise a nonwoven, which may be hydrophilic. One or both of upper and lower acquisition layers 52 and 54 may comprise chemically cross-linked cellulosic fibers, which may or may not form part of a nonwoven material. Upper acquisition layer 52 may comprise a nonwoven, without cross-linked cellulosic fibers, and lower acquisition layer 54 may comprise chemically cross-linked cellulosic fibers. Lower acquisition layer 54 may comprise chemically cross-linked cellulosic fibers mixed with other fibers such as natural or synthetic polymeric fibers. Such other natural or synthetic polymeric fibers may include high surface area fibers, thermoplastic binding fibers, polyethylene fibers, polypropylene fibers, PET fibers, rayon fibers, lyocell fibers, and mixtures thereof.

Exemplary nonwoven materials for upper and lower acquisition layers 52 and 54 include, but are not limited to, SMS material comprising a spunbonded layer, a melt-blown layer, and a further spunbonded layer, and variations on an SMS material, such as SMMS, SSMMS, SSMMMS, SMMMS, or SSMMXS material, where X can be a meltblown layer or a spunbonded layer. In some embodiments, permanently hydrophilic nonwovens, such as nonwovens with a durable hydrophilic coating, may be desirable. In some embodiments, the upper and/or lower acquisition layers 52 and 54 may comprise an SSMMS-structure. In some embodiments, the nonwovens may be porous.

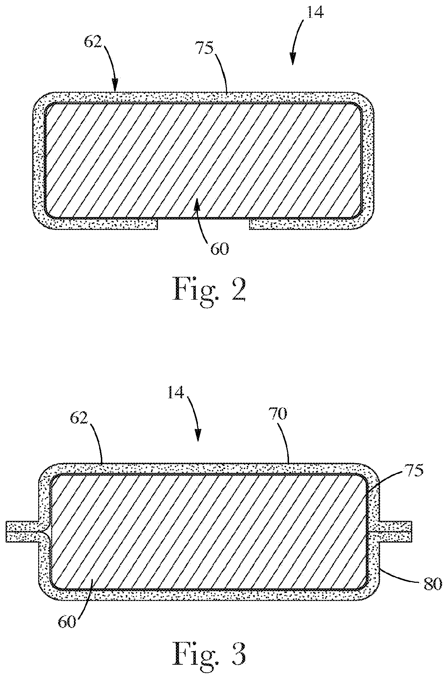

Fig. 2 is a sectional view along 2-2 showing a section of one embodiment of an absorbent core 14 of diaper 10. In Fig. 2 absorbent core 14 comprises storage layer 60, which is wrapped by core wrap 62 in a C-fold. Core wrap 62 may comprise a substrate which has been coated with a durable hydrophilic coating composition. The substrate may be a nonwoven, a polymeric film, or combinations thereof. In some embodiments, core wrap 62 functions as an acquisition layer, making separate upper and lower acquisition layers 52 and 54 unnecessary.

Fig. 3 is a sectional view along 2-2 showing a section of an alternate embodiment of absorbent core 14 of diaper 10. In Fig. 3 storage layer 60 is surrounded or enveloped by core wrap 62. Core wrap 62 comprises fluid receiving top layer or substrate 70 and bottom layer or substrate 80. Fluid receiving top layer 70 and bottom layer 80 can be the same or different. That is, they may be the same substrate coated with the same durable hydrophilic coating composition, different substrates coated with the same durable hydrophilic coating composition, or different substrates coated with different durable hydrophilic coating compositions. In some embodiments, bottom layer 80 is not coated. Top layer 70 of storage layer 60 may be referred to as a core cover and bottom layer 80 of storage layer 60 may be referred to as a dusting layer. In some embodiments, top layer 70 and bottom layer 80 may comprise nonwoven material. One exemplary material is an SMS material, comprising a spunbonded, a melt-blown, and a further spunbonded layer, and variations on SMS, such as SMMS or SSMMS. Top layer 70 and bottom layer 80 may be provided from two or more separate sheets of nonwoven materials, or they may be formed from a unitary sheet of material.

The structures of diaper 10 which may be desirably hydrophilic include, but are not limited to, topsheet 18, absorbent core wrap 62, and upper and lower acquisition layers 52 and 54. In some embodiments, only one of top layer 70 or bottom layer 80 of core wrap 62 is hydrophilic. For example, top layer 70 may be hydrophilic and bottom layer 80 may be hydrophobic, or top layer 70 may be treated to render it hydrophilic and bottom layer 80 may be untreated. In various diaper configurations it may be desirable to have a hydrophilic nonwoven in one of these structures, all of these structures, or other structures, and the durably hydrophilic coated nonwoven of the present disclosure may be suitable for use in any such structure. Further, in some structures it may be desirable for only part of a structure or component to be hydrophilic. For example, it may be desirable for topsheet 18 to be hydrophilic in a transversely central portion of crotch region 34, and for transversely outboard portions of topsheet 18 to be hydrophobic. Where desired, only a portion of a structure or component may be coated or treated to confer hydrophilic properties.

METHOD OF REDUCING MIGRATION

The structures and compounds described above may be used to reduce the migration of a hydrophilic coating to adjacent structures in an absorbent article. For example, the migration of a hydrophilic coating from one structure in an absorbent article to an adjacent structure or structures in an absorbent article may be reduced by providing a first hydrophobic nonwoven substrate, coating the first hydrophobic nonwoven substrate with a coating composition comprising a polyether-modified trialkoxysilane to produce a coated nonwoven substrate, and incorporating the coated nonwoven substrate into an absorbent article. The coated nonwoven substrate may be placed adjacent to a second hydrophobic nonwoven substrate. The second hydrophobic nonwoven substrate may be inherently hydrophobic or may have been treated to make the nonwoven hydrophobic or to increase its hydrophobicity. The absorbent article may comprise at least a trace amount of an oleaginous substance, as described above.

The coated nonwoven substrate may be adjacent to the oleaginous substance, including positioning the coated nonwoven substrate on the oleaginous substance or vice versa. For example, an adhesive containing the oleaginous substance may be disposed near the coated nonwoven substrate or disposed on the coated nonwoven substrate. As a further example, an adhesive or elastic member containing the oleaginous substance may be disposed in between the coated nonwoven substrate and the second hydrophobic nonwoven substrate. In some embodiments, an adhesive containing an oleaginous substance may be used to join the coated nonwoven substrate to the second hydrophobic nonwoven substrate in the absorbent article.

EXAMPLES

Example 1

An aminopolyether-modified trialkoxysilane coating solution is prepared as follows: The aminopolyether-modified trialkylsiloxane is dispersed in water at the desired concentration. If a process aid such as defoamer or wetting agent is desired, these are dispersed next. With stirring, citric (reference) or acetic acid (according to the invention) is next dissolved into the mixture, if desired.

Exemplary Coating Solutions by Composition, weight of component to weight of solution

| Example → Components↓ | 2 | 3 | 4 | 5 | 6 | 7 | 8 | 9 | 10 | 11 | 12 | 13 | 14 | 15 | 16 |
|---|---|---|---|---|---|---|---|---|---|---|---|---|---|---|---|
| A | 7.5 | 7.5 | 12 | 15 | 20 | 20 | 20 | 10 | 10 | 10 | 10.1 | 10 | 10 | 8.9 | 11.5 |
| B | 0 | 0 | 0 | 0 | 0 | 0 | 0 | 0.5 | 0.1 | 1 | 0.6 | 0.3 | 0.5 | 0.9 | 0.5 |
| C | 0 | 0 | 0 | 0 | 0 | 0.75 | 2 | 1.5 | 1.5 | 1 | 0.7 | 0.7 | 0 | 0 | 0 |
| D | 0 | 0 | 0 | 0 | 0 | 0 | 0.9 | 1.8 | 2 | 1.7 | 1.4 | 1.5 | 1.5 | 1.3 | 1.2 |
| E | 0 | 1.6 | 1.56 | 2.9 | 0.95 | 1.25 | 0 | 0 | 0 | 0 | 0 | 0 | 0 | 0 | 0 |

A is an aminopolyether-modified trialkoxysilane available from Momentive Performance Materials of Albany, NY, USA as Y17219.
B is a wetting agent available from Momentive Performance Materials of Albany, NY, USA as Y17309.
C is a silicone surfactant available from Momentive Performance Materials of Albany, NY, USA as NuWet™ 550.
D is citric acid.
E is acetic acid.

Example 17

A 10 gsm SSMMS substrate formed of polypropylene fibers is coated with the solution of Example 16 by kissrolling. The coating solution is applied over the entire surface at 1.3g/m$^2$ of substrate surface, and then dried at ~100°C for 0.5 seconds to 40 seconds, or until the sample is sufficiently dry for further handling or for its intended use. The coated nonwoven of example 16 is then used as a core wrap in an absorbent article as shown in Figures 1 and 2.

TEST METHODS

Wettability

Laminate Preparation:

Samples are prepared in a lab controlled at 23°C ± 2C° and relative humidity of 50% ± 2%. The oven used for conditioning the samples is capable of maintaining a temperature of 60°C ± 2C° over a period of 7 days. The analyst must wear nitrile disposable gloves while handling the absorbent article and any pieces to avoid contamination of the test specimen.

Wettability is tested on core cover, dusting layer, and top sheet materials harvested from an absorbent article. If necessary, pieces from multiple absorbent articles can be used to construct a single laminate. Only a single type of test material (i.e., topsheet, dusting layer, or core cover) is incorporated into an individual laminate.

The test material is cut into two squares, 75 mm by 75 mm, avoiding any edge seams that were mechanically or adhesively bonded in the product. Note which side faced the interior of the core. Taking one piece of test material, apply 10 gsm of Fabu-Las adhesive (commercially available from H.B. Fuller, St. Paul, MN) in two parallel rows by slot coating to the side facing the interior of the absorbent core. The rows are spaced 25 mm apart and 25 mm from the parallel sides of the test material. Taking a matching piece of the same material, carefully place the side that was facing the interior of the absorbent core onto the glue layer, aligning all four edges and ensuring no wrinkling. A 2 kg hand-held roller is then rolled 2 times (i.e., once forward then back) over the laminate to ensure good nonwoven-to-adhesive bonding. This procedure is repeated for 3 laminates of each test material.

Each laminate is then individually wrapped in aluminum foil and placed into the oven set at 60°C. A 5.5 Kg weight, with a length and width measuring 75 mm is centered on each individual laminates and aged for 168 hours (7 days). After aging, the samples are removed from the oven and allowed to cool to room temperature while still in the aluminum foil. If necessary, a cryogenic spray, such as Cyto-Freeze (obtained from Control Company, Houston, TX), may be used to remove the laminate from the foil by spraying the outside of the foil.

Wettability Test:

A round dish 15 mm deep by 150 mm in diameter is filled to 1 cm with deionized water. Gently lay the laminate on the surface of the water. The bottom layer of the laminate is assumed to wet during the course of the test. Observations regarding top layer wetting uniformity and speed are observed and recorded. A number value is then assigned using the following scale:

1 = Top layer wets immediately and completely
2 = After 30 seconds the top layer has wet completely, but was not immediate
3 = After 30 seconds most of top layer is wetted
4 = After 30 seconds the top layer is only partially wetted
5 = After 30 seconds the top layer is still dry

The test is performed on three replicates of the laminate that were prepared and their score averaged. The answer is reported out to 0.1 unit. Testing is performed in like fashion for each type of test material: topsheet, core cover and dusting layer.

Permittivity ($\varepsilon''$)

The mass of hydrophilic coating residue that migrates to the absorbent article's cuff is measured using an Impedance Analyzer (Agilent RF Impedance Analyzer E4991A with E4991A option 010 test head or equivalent). The permittivity of an aqueous extract of the cuff is measured and the concentration of residue is determined via correlation of the permittivity parameter $\varepsilon''$ verses standard solutions.

Instrument Setup and Calibration

The Impedance Analyzer is maintained and operated as per the manufacturer's instructions. For this analysis, it is programmed to scan a frequency range from 1.0 MHz to 1.5 MHz using the log frequency sweep setting assuring greater than 8 points are taken across the frequency range. All experiments are conducted at 23°C $\pm$ 2C°.

The instrument is calibrated against a Silastol PHP26 standard material available from Schill & Seilacher (Böblingen Germany). All standards and samples are prepared with purified deionized (18 MΩ) water, such as water from a Barnstead Nanopure Type I ultrapure water system (Thermo Fisher Scientific, USA, or equivalent). Six standards are prepared consisting of 10 μg, 20 μg, 40 μg, 60 μg, 80 μg, and 100 μg each dissolved into 35.0 mL $\pm$ 0.1 mL of deionized water. Weights are recorded to the nearest 1 μg. Samples are prepared in separate 50 mL screw top polypropylene centrifuge tubes (VWR SuperClear or equivalent). At the same time prepare a blank deionized water reference from the same deionized water source.

Permittivity measurement of a specimen is conducted by immersing the test head probe into the deionized water solution, initiating a scan, and collecting $\varepsilon''$ data between 1.0 MHz and 1.5 MHz. All measured $\varepsilon''$ values collected over the specified frequency range are averaged to obtain an average $\varepsilon''$ value and reported to the nearest 1 unit. This procedure is repeated for each standard and blank. The blank-corrected $\varepsilon''$ values for the standards are calculated by subtracting the $\varepsilon''$ value of the blank from the $\varepsilon''$ value of each standard. A linear, least squares, calibration plot is constructed from the blank-corrected $\varepsilon''$ values versus concentration (μg/35 mL) of Silastol PHP26.

Sample Measurements

The full lengths of the inner cuffs are removed from the product, removing any and all attached topsheet, backsheet film, and other similar materials from the cuff. Cryogenic spray such as Cyto-Freeze (Control Co., Houston TX) can be used to facilitate as necessary. Adhesives used to attach the cuff to the chassis may remain on the cuff. The cuff is fully extended flat on a lab bench and the width and length are measured to the nearest 1 mm. The length and width are then multiplied to calculate the area of the cuff and is recorded to the nearest 1 mm$^2$. Each cuff is placed into a separate centrifuge tube and 35.0 mL $\pm$ 0.1 mL of deionized water is added to each centrifuge tube. Assure the cuff is totally submerged into the deionized water (if necessary minimally compress the cuff such that it is below the surface). At the same time prepare a blank deionized water reference from the same deionized water source. The samples are soaked for 12 hours then hand mixed by vigorous shaking for 1 min. immediately before analysis. The cuff material is left in the centrifuge tube as the measurement is conducted.

Analyze each sample in like fashion to the calibration standards as described above. The measurement of each sample and blank is conducted by immersing the test head probe into the deionized water solution, initiating a scan, and collecting ε" data between 1.0 MHz and 1.5 MHz. All measured ε" values collected over the specified frequency scan are averaged to obtain an average ε" value and recorded to the nearest 1 unit. This procedure is repeated for each sample and blank. The blank-corrected ε" values for the samples are calculated by subtracting the ε" value of the blank from the ε" value of each sample. Using the calibration curve, determine the micrograms of residue present in each sample from the measured blank-corrected ε" value.

$$\text{Residue on Cuff (g/m}^2) = \text{ residue (µg) / cuff area (mm}^2) \text{ x } (10^6 \text{ mm}^2/\text{m}^2) \text{ x } (\text{g}/10^6\text{µg})$$

Time-of-Flight Secondary Ion Mass Spectrometry (ToF-SIMS)

Investigation of the surface chemistry of solid materials, including, but not limited to nonwovens, can be accomplished by using time-of-flight secondary ion mass spectrometry (ToF-SIMS). ToF-SIMS utilizes a focused, pulsed, energetic ion beam (referred to as "primary ions") to sputter material from the sample's surface. Some of the sputtered material is ionized (referred to as "secondary ions"), and is mass-analyzed and detected in a time-of-flight mass spectrometer. Sample damage is minimized by maintaining a primary ion dose density below the static limit of $10^{12}$ primary ions/cm$^2$ (referred to as the "static limit"). For electrically insulating samples such as nonwovens, charge compensation is accomplished using a pulsed, low kinetic energy electron flood gun.

Instrument Parameters

In this method, a time-of-flight secondary ion mass spectrometer equipped with a primary ion source selected from one of the following: $Cs^+$, $Au_1^+$, $Au_3^+$, $Bi_1^+$, $Bi_3^+$, or $C_{60}^+$ in the kinetic energy range including 8 keV to 25 keV, a pulsed low kinetic energy electron flood gun, and a time-of-flight mass spectrometer including, but not limited to, a single-stage reflectron time-of-flight mass spectrometer is used. Post-acceleration of the secondary ions in the mass spectrometer using a 10 kV post-acceleration field should be used before the secondary ions impact the detector. One such ToF-SIMS instrument that meets these requirements is the ToF-SIMS IV instrument made by Ion-Tof GmbH (Munster, Germany), though other ToF-SIMS instruments may also meet these requirements.

The angle of incidence of the primary ion beam to the sample (defined as the angle between the ion beam and the sample normal) should be between 40 degrees and 50 degrees. The repetition rate of the primary ion beam should be between 5 kHz and 10 kHz. The primary ion beam should be rastered over the analysis area using a random raster. The raster area of the primary ion beam should be between 300 µm x 300 µm to 500 µm x 500 µm using a pixel density of 128 pixels x 128 pixels. The pulse duration of the primary ion beam must be below 2 nanoseconds, as determined by the full width at half maximum (FWHM) of the $H^+$ peak in a positive ion mass spectrum of a clean silicon wafer sample acquired under the same instrumental conditions as used to analyze the test samples. The pulsed primary ion current must be below 2 pA as measured using a Faraday cup under the same primary ion beam conditions as used to analyze the test sample. Both positive ion mode and negative ion mode spectra can be acquired, as long as the total primary ion dose density applied in each raster field is below the static limit of $10^{12}$ primary ions/cm$^2$. No additional ion beams other than the primary ion source should be used during the analysis. The kinetic energy of the electrons generated by the electron flood gun used for charge compensation must be below 25 eV. The current of the electron flood gun used for charge compensation must be between 10 µA and 25 µA. The pressure in the analytical chamber of the ToF-SIMS instrument must be below 3 x $10^{-11}$ bar during the analysis of the test samples.

<u>Sample Preparation for ToF-SIMS Analysis</u>

Samples are prepared in a lab controlled at 23°C $\pm$ 2C° and relative humidity of 50% $\pm$ 2%. The oven used for conditioning the samples is capable of maintaining a temperature of 60°C $\pm$ 2C° over a period of 7 days. Sample handling prior to ToF-SIMS analysis should conform to ASTM Standard E1829-09 ("Standard Guide to Handling Specimens Prior to Surface Analysis").

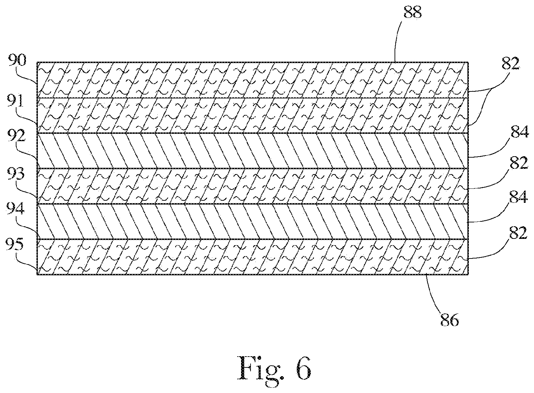

Individual sample stacks for topsheet, dusting layer, and core cover are prepared for ToF-SIMS analysis. These three sample stacks are constructed from materials harvested from absorbent products combined with a representative cuff material (uncoated 15 gsm SMS spunmelt nonwoven commercially available from First Quality Nonwovens, McElhattan, PA), combined as depicted in Figure 6. From top 88 to bottom 86, the stacks have two layers 90, 91 of test material 82, a layer 92 of uncoated nonwoven 84, another layer 93 of test material 82, another layer 94 of uncoated nonwoven 84, and a final layer 95 of test material 82. Only a single type of test material is incorporated into an individual stack. If a single absorbent article is not large enough to harvest enough pieces for one stack, matching pieces from multiple absorbent articles can be combined to construct a stack.

A test nonwoven material (i.e., topsheet, dusting layer, or core cover) is harvested from an absorbent article, noting which side would face the wearer's body. This material is then cut into four squares 75 mm by 75 mm, avoiding any edge seams that were mechanically or adhesively bonded in the product. Referring to Figure 6, the orientation of the layers are as follows: layer 90 and 91 have their body facing sides directed toward layer 92, while layers 93 and 95 have their body facing sides directed toward layer 94. Each stack is then wrapped in aluminum foil and placed into the oven set at 60°C. A 5.5 Kg weight, with a length and width measuring 75 mm is centered on each individual sample stack and aged for 168 hours (7 days). After aging, the samples are removed from the oven and must be analyzed using ToF-SIMS within one week.

Each stack is taken from the oven and the aluminum foil removed. Carefully separate the individual layers from each other, keeping track of their orientation. Using a scalpel, dissect two, 1 cm by 2 cm specimens from each layer. The locations should be selected randomly, but can not be within 15 mm of each other or within 15 mm of the edge of the sample. Immediately before mounting, cut the specimen in half to yield two 1 cm square pieces. One half will be tested on its top side (with respect to the stack) the other half being tested on its bottom side (once again with respect to the stack). This procedure is repeated for each aged stack.

Data Acquisition

For analysis, the 1 cm$^2$ specimen is mounted onto an aluminum or steel sample stub using double-sided adhesive tape. Once mounted, a specimen is introduced into the ToF-SIMS instrument for analysis under the conditions described above. Positive ion mode spectra on at least five randomly-spaced, non-overlapping raster fields should be acquired on each sample. This procedure is repeated for each top/bottom pair and for layers of the stack being analyzed.

Data Analysis

The data obtained from stacks of each test material (topsheet, core cover, dusting layer) are analyzed separately. Each mass spectrum should be mass calibrated before further analysis. Positive ion mass spectra are mass calibrated using the $CH_3^+$ (nominal m/z=15), $C_2H_3^+$ (nominal m/z = 27), and $C_3H_5^+$ (nominal m/z = 41) peaks. Only positive ion mode mass spectra are required for this analysis. After mass calibrating the spectra, the intensities in each spectrum at nominal m/z = 41 (integrating from m/z = 40.75 to m/z = 41.75), nominal m/z = 45 (integrating from m/z = 44.75 to m/z = 45.75), and nominal m/z = 59 (integrating from m/z = 58.75 to m/z = 59.75) are integrated and tabulated. Without impacting the results of this analysis, nominal m/z = 41 is assigned to $C_3H_5^+$, nominal m/z = 45 is assigned to $C_4H_5O^+$, and nominal m/z = 59 is assigned to m/z = $C_5H_7O^+$. The oxygen-containing secondary ions $C_4H_5O^+$ and $C_5H_7O^+$ provide an indicator for the poly(ethylene oxide) and poly(propylene oxide) portions of the coating, respectively. For each mass spectrum, the intensity of the $C_4H_5O^+$ peak ($I_{C4H5O+}$) and the intensity of the $C_5H_7O^+$ peak ($I_{C5H7O+}$) are summed together. This summed intensity for each spectrum is then divided by the intensity of $C_3H_5^+$ peak ($I_{C3H5+}$) for each spectrum to correct for variations in secondary ion yield between raster areas on the samples. This peak intensity ratio is then defined as the "Surface Modification Ratio", SMR, for each spectrum:

$$SMR(i) = \frac{I_{C4H5O+}(i) + I_{C5H7O+}(i)}{I_{C3H5+}(i)} \qquad (Eq.\ 1)$$

The average SMR for all of the spectra (top side and bottom side) for the coated test material layers in the stack of nonwoven materials (layers 90, 91, 93, and 95) is then calculated to determine the "Average Treated SMR", ATSMR. All SMR values for all of the spectra acquired for the nonwoven stack are then divided by the ATSMR to generate the "Normalized SMR", NSMR:

$$NSMR(i) = \frac{SMR(i)}{ATSMR} \qquad (Eq.\ 2)$$

The 10 NSMR values obtained from the same side of each of the six layers are then averaged and reported.

The ToF-SIMS method can also be performed on raw materials (that is, materials which have not been assembled into an absorbent article), as in the following examples.

*Example 18*

The average NSMR values for each of the layers of a stack consisting of coated nonwoven core covers (10 gsm SSMMS spunmelt nonwoven available from Fibertex Nonwovens, Denmark) as layers 90, 91, 93, and 95 and uncoated nonwoven cuff materials (15 gsm SMS spunmelt nonwoven commercially available from First Quality Nonwovens) as layers 92 and 94 are shown in Table 1 below. The low NSMR values for the untreated nonwoven cuff materials (layers 92 and 94) demonstrate that there was little to no migration of the surface treatment from the coated nonwoven layers onto the uncoated nonwoven layers.

| Table 1. NSMR Values for Example 18 | | |
|---|---|---|
| | NSMR Values (Average $\pm$ Standard Deviation) | |
| Layer | (Top Side) | (Bottom Side) |
| 90: Test Material | 0.9 $\pm$ 0.1 | 1.1 $\pm$ 0.1 |
| 91: Test Material | 1.0 $\pm$ 0.1 | 1.1 $\pm$ 0.1 |
| 92: Uncoated Nonwoven | 0.0 $\pm$ 0.0 | 0.0 $\pm$ 0.0 |
| 93: Test Material | 1.1 $\pm$ 0.1 | 0.8 $\pm$ 0.3 |
| 94: Uncoated Nonwoven | 0.0 $\pm$ 0.0 | 0.0 $\pm$ 0.0 |
| 95: Test Material | 0.9 $\pm$ 0.2 | 1.1 $\pm$ 0.1 |

*Example 19*

The average NSMR values for each of the layers of a stack consisting of coated nonwoven core covers (10 gsm SSMMS spunmelt nonwoven) as layers 90, 91, 93, and 95 and uncoated nonwoven cuff materials (15 gsm SMS spunmelt nonwoven commercially available from First Quality Nonwovens) as layers 92 and 94 are shown in Table 2 below. The low NSMR values for the uncoated nonwoven cuff materials (layers 92 and 94) demonstrate that there was little to no migration of the surface treatment from the coated nonwoven layers onto the uncoated nonwoven layers.

| Table 2. NSMR Values for Example 19 | | |
|---|---|---|
| | NSMR Values (Average $\pm$ Standard Deviation) | |
| Layer | (Top Side) | (Bottom Side) |
| 90: Test Material | 0.8 $\pm$ 0.3 | 0.9 $\pm$ 0.2 |
| 91: Test Material | 1.0 $\pm$ 0.2 | 1.0 $\pm$ 0.2 |
| 92: Uncoated Nonwoven | 0.0 $\pm$ 0.0 | 0.0 $\pm$ 0.0 |
| 93: Test Material | 1.0 $\pm$ 0.1 | 1.1 $\pm$ 0.0 |
| 94: Uncoated Nonwoven | 0.0 $\pm$ 0.0 | 0.0 $\pm$ 0.0 |
| 95: Test Material | 1.2 $\pm$ 0.0 | 1.0 $\pm$ .03 |

*Example 20*

The average NSMR values for the each of the layers of a stack consisting of coated nonwoven dusting layers (11 gsm SSMMS spunmelt nonwoven) as layers 90, 91, 93, and 95and uncoated nonwoven cuff materials (15 gsm SMS spunmelt nonwoven commercially available from First Quality Nonwovens) as layers 92 and 94 are shown in Table 3 below. The low NSMR values for the uncoated nonwoven cuff materials (layers 92 and 94) demonstrate that there was little to no migration of the surface treatment from the coated nonwoven layers onto the uncoated nonwoven layers.

| Table 3. NSMR Values for Example 20 | | |
|---|---|---|
| | NSMR Values (Average ± Standard Deviation) | |
| Layer | (Top Side) | (Bottom Side) |
| 90: Test Material | 0.9 ± 0.5 | 1.0 ± 0.2 |
| 91: Test Material | 1.0 ± 0.3 | 1.0 ± 0.3 |
| 92: Uncoated Nonwoven | 0.0 ± 0.0 | 0.0 ± 0.0 |
| 93: Test Material | 0.9 ± 0.2 | 1.0 ± 0.2 |
| 94: Uncoated Nonwoven | 0.0 ± 0.0 | 0.1 ± 0.0 |
| 95: Test Material | 1.0 ± 0.4 | 1.2 ± 0.1 |

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

**Claims**

1. An absorbent article comprising:

   a mineral oil;
   a hydrophobic nonwoven substrate disposed adjacent to the mineral oil; and
   a coating composition comprising a polyether-modified trialkoxysilane disposed on the nonwoven substrate, a wetting agent and citric acid

   wherein the coated nonwoven substrate has a Residue on Cuff value less than 0.002 $g/m^2$ and wherein the polyether moiety of the amino-polyether-modified trialkoxysilane has an average structure $CH_2(O)CHCH_2(OCH_2CH_2)_{6.9}OCH_2CH(O)CH_2$ and wherein the mineral oil is a component of a hot melt adhesive.

2. The absorbent article of Claim 1, wherein the coating is applied to the nonwoven substrate in an amount of at least 0.1 % by weight of the nonwoven.

3. The absorbent article of Claim 1, wherein the nonwoven substrate is a component of a structure selected from the group consisting of a topsheet, an upper acquisition layer, a lower acquisition layer, a core wrap, a core cover, and a dusting layer.

**Patentansprüche**

1. Absorptionsartikel, umfassend:

   ein Mineralöl;
   ein hydrophobes an das Mineralöl angrenzendes Vliessubstrat; und
   eine Beschichtungszusammensetzung, umfassend ein Polyethermodifiziertes Trialkoxysilan, angeordnet auf dem Vliessubstrat; ein Benetzungsmittel und Citronensäure

wobei das beschichtete Vliessubstrat einen Residue-on-Cuff-Wert von 0,002 g/m$^2$ und wobei die Polyether-Einheit des Amin-Polyether-modifizierten Trialkoxysilans eine durchschnittliche Struktur $CH_2(O)CHCH_2(OCH_2CH_2)_{6,9}OCH_2CH(O)CH_2$ aufweist, und wobei das Mineralöl ein Bestandteil eines Hotmelt-Klebstoffs ist.

2. Absorptionsartikel nach Anspruch 1, wobei die Beschichtung mit einer Menge von 0,1 Gew.-% des Vlieses auf das Vliessubstrat aufgetragen wird.

3. Absorptionsartikel nach Anspruch 1, wobei das Vliessubstrat ein Bestandteil einer Struktur ist, ausgewählt aus der Gruppe bestehend aus einer Deckschicht, einer oberen Aufnahmeschicht, einer unteren Aufnahmeschicht, einer Kernumwicklung, einer Kernabdeckung und einer Stäubeschicht.

**Revendications**

1. Article absorbant comprenant :

une huile minérale ;

un substrat non tissé hydrophobe disposé adjacent à l'huile minérale ; et

une composition de revêtement comprenant un trialcoxysilane à modification polyéther disposé sur le substrat non tissé ; un agent mouillant et de l'acide citrique

dans lequel le substrat non tissé revêtu a une valeur de résidu sur revers inférieure à 0,002 g/m$^2$ et dans lequel le fragment polyéther du trialcoxysilane à modification aminopolyéther a une structure moyenne $CH_2(O)CHCH_2(OCH_2CH_2)_{6,9}OCH_2CH(O)CH_2$ et dans lequel l'huile minérale est un composant d'un adhésif thermofusible.

2. Article absorbant selon la revendication 1, dans lequel le revêtement est appliqué au substrat non tissé en une quantité d'au moins 0,1 % en poids du non-tissé.

3. Article absorbant selon la revendication 1, dans lequel le substrat non tissé est un composant d'une structure choisie dans le groupe constitué d'une feuille de dessus, d'une couche de recueil supérieure, d'une couche de recueil inférieure, d'une enveloppe d'âme, d'une protection d'âme et d'une couche de saupoudrage.

Fig. 1

Fig. 2

Fig. 3

Fig. 4

300

330

360    350    360    360    370

310    320

Fig. 5

Fig. 6

Fig. 7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2009117793 A
- US 5246433 A
- US 5569234 A
- US 6120487 A
- US 6120489 A
- US 4940464 A
- US 5092861 A
- US 20030233082 A1
- US 5897545 A
- US 5957908 A
- US 20040167486 A
- US 20060021695 A
- US 20080125735 A
- US 20090117793 A, Falk
- US 20090118421 A, Falk
- US 3860003 A
- US 5151092 A
- US 5554145 A
- US 6004306 A
- US 5137537 A

**Non-patent literature cited in the description**

- Contact angle, wettability and adhesion. *American Chemical Society,* 1964